# EUROPEAN PATENT APPLICATION

(11) **EP 2 633 820 A1**
(43) Date of publication of application: **04.09.2013**
(21) Application number: 12157605.2
(22) Date of filing: 29.02.2012
(51) Int. Cl.: A61B 17/00

(54) **A medical implant for occluding an opening in a body and a method of producing such a medical implant**

(71) Applicant: Occlutech Holding AG, 8201 Schaffhausen (CH)
(72) Inventor: Ottma, Rüdiger, 99441 Großschwabhausen (DE); Tilchner, Sebastian, 07743 Jena (DE)
(74) Representative: KIPA AB

(57) **Abstract**

The disclosure relates to a medical implant for occluding an opening in a body and a method of producing such a medical implant. Disclosed is an improved occluder, which provides improved occlusion, improved sealing and improved endothelialization. The occluder slows down the blood flow through the defect. In one embodiment, a medical implant 1 for occluding an opening in a body is provided, wherein the medical implant 1 comprises a body mesh of strands forming a plurality of adjacent cells delimited by the strands, the body mesh having an external surface, and a coating 22 or a non-fibrous film membrane 17 covering the external surface for at least partly restricting a fluid flow through a structural tissue defect, such as a defect in the heart.

## Description

### BACKGROUND OF THE INVENTION

### Field of the Disclosure

This disclosure pertains in general to the field of medical implants. More particularly, the disclosure relates to occluding devices or occluders. In particular the disclosure relates to applying of a coating to an external surface of a medical implant or to coverage of an outside of a medical implant with a coating or a non-fibrous film membrane for improving the sealing of a defect, such as a defect in the heart.

### Description of the Prior Art

An occluder is a medical product or implant used for occluding, i.e. closing, defects e.g. in the human heart. Defects may occur in various regions of the heart and have different forms. Defects in the septum of the atrium are common. The occluders can be inserted using minimally invasive cardiac catheter techniques, more precisely by means of a transvenous, catheter-interventional access. One type of occluders is made of Nitinol threads, e.g. having a double disc shape with an intermediate tubular section, having a smaller diameter than the discs, between the discs. They are inserted in openings that are to be closed, one disc on each side of the hole, which is to be closed and with the intermediate tubular section in the center of the hole, the discs being larger than the hole. There are two examples of such devices. The first, made by Occlutech^{®,} having one fixation point at the end of the device and the second, made by AGA medical^{®} having two fixation points, one at each end of the device. In these devices, the Nitinol threads are joined in the centre of one or both of the discs.

Some of the fixation points have a screw with windings to be attached to a rod. By means of that rod, the devices may be pulled into and pushed out of a catheter when being positioned in the opening to be closed. When in position, the device is detached from the rod by unscrewing the connection.

These occluders normally comprise some kind of mesh and may be provided with a polyester fabric inside the mesh for limiting the blood flow through the occluder. An example of such a prior art polyester fabric is given in the published US application US2009/0082803 A1.

However, an occluder having an internal fabric may be difficult and time-consuming to produce. Moreover, the use of artificial fibrous material in an occluder may lead to blood clotting or thrombosis. If the clotting is too severe and the clot breaks free, the clot may travel to other parts of the body. Such travelling clots are known as embolus. Emboli are carried by the circulation and capable of clogging arterial capillary beds at a site different from its point of origin, i.e. they can create an unwanted arterial occlusion or vascular occlusion. When vascular occlusion occurs in a major vein, it can cause deep vein thrombosis. Deep vein thrombosis commonly affects the leg veins, e.g. the femoral vein or the popliteal vein, or the veins in the pelvis. The most serious complication of a deep vein thrombosis is that the clot could dislodge and travel to the lungs, which is called a pulmonary embolism. There, in the lungs, the clots can block the main artery of the lungs. The consequences of such a blockage may be severe.

Thus, it would be advantageous to provide blood flow limiting means other than an internal fibrous fabric.

### SUMMARY OF THE INVENTION

Accordingly, embodiments of the present disclosure preferably seek to mitigate, alleviate or eliminate one or more deficiencies, disadvantages or issues in the art, such as the above-identified, singly or in any combination by providing a medical implant for occluding an opening in a body and a method of producing such a medical implant, according to the appended patent claims.

According to aspects of the disclosure, a medical implant and a method of producing such a medical implant are disclosed, wherein the medical implant is covered on an outside with a coating or a non-fibrous film membrane for sealing of a defect, such as a defect in the heart.

According to one aspect of the disclosure, a medical implant, such as an atrial septal defect (ASD) occluder, a Patent foramen ovale (PFO) occluder, a paravalvular leakage (PLD) occluder or a ventricular septal defect (VSD) occluder, for occluding an opening in a body is provided. The medical implant comprises a body mesh of strands forming a plurality of adjacent cells delimited by the strands. The body mesh has an external surface and may be made of strands, which has been e.g. braided, knitted or woven together to form the mesh. A coating or a non-fibrous film membrane covers the external surface for at least partly restricting a fluid flow through a structural tissue defect, such as a defect in the heart.

According to another aspect of the disclosure, a method of producing a medical implant for occluding an opening in a body is provided. The method comprises producing a body mesh of strands forming a plurality of adjacent cells delimited by the strands. It further comprises applying a polymer to at least part of an external surface of the medical implant. The polymer is applied to the medical implant by dipping, spraying, electro-spinning, electro-spraying, Nano-spinning or by sewing a non-fibrous film membrane onto the external surface of the medical implant.

Further embodiments of the disclosure are defined in the dependent claims, wherein features for the second and any other aspects of the disclosure are as for the first aspect mutatis mutandis.

The use of a coating outside an external surface of a medical implant provides for a lower friction of the medical implant in e.g. a catheter.

Some embodiments of the disclosure also provide for an improved occlusion.

Some embodiments of the disclosure also provide for improved sealing of a defect, such as a heart defect.

Some embodiments of the disclosure also provide for an improved endothelialization.

Some embodiments of the disclosure also provide for slowing down the blood flow through the defect.

Some embodiments of the disclosure also provide for an advantageous and/or easier delivery of the medical implant, since the use of a coating outside an external surface of a medical implant may make the medical implant glide or slide easier through a delivery catheter.

Some embodiments of the disclosure also provide for enabling an initial controllable fluid retention.

Some embodiments of the disclosure also provide for that an inflow of blood to different areas of a medical implant is controlled or controllable.

Some embodiments of the disclosure also provide for that the flow is efficiently restricted by covering at least substantially the full diameter of both ends of the medical implant.

Some embodiments of the disclosure also provide for that integration of the medical implant with surrounding blood is enhanced.

Some embodiments of the disclosure also provide for that the coating or the non-fibrous membrane is free of tension, so that pre-mature fatigue thereof can be avoided and thus a reliable ingrowth is allowed for.

Some embodiments of the disclosure also contribute to facilitation of expansion into an expanded shape, since the coating elastically contributes to expansion into the expanded shape, i.e. by making the coating elastic and by applying the coating to the medical implant, while the medical implant is in its expanded shape, the coating on the external surface of the medical implant is prone to contribute to force the medical implant into its expanded shape.

Some embodiments of the disclosure also provide for facilitation of the delivery of the medical implant through a catheter, since the coating is prone to contribute to force the medical implant into its contracted shape if the coating is applied to the medical implant while the medical implant is in its contracted shape.

Some embodiments of the disclosure also provide for that the occlusion is not abrupt upon implantation.

Some embodiments of the disclosure also provide for that a certain blood flow may still occur after implantation and gradually decline upon blood coagulation and/or endothelialization of the implanted medical implant.

Some embodiments of the disclosure also provide for that friction of the medical device is lowered, e.g. during delivery through a catheter.

Some embodiments of the disclosure also provide for that cellular biocompatibility is maximized.

Some embodiments of the disclosure also provide for a medical implant, which is easier and cheaper to manufacture than a medical implant having patches inside, since no sewing is necessary.

Some embodiments of the disclosure also provide for a less time consuming manufacturing of a medical implant.

Some embodiments of the disclosure also provide for a very flexible medical implant.

Some embodiments of the disclosure also provide for a medical implant with a particularly large expansion/contraction ratio.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the disclosure are capable of will be apparent and elucidated from the following description of embodiments of the present disclosure, reference being made to the accompanying drawings, in which
Fig. 1a is a lateral view of a medical implant with a coating outside the medical implant;
Fig. 1b is a lateral view of a medical implant with a non-fibrous membrane outside the medical implant;
Fig. 2a is a top view of a medical implant;
Fig. 2b is a top view of a medical implant having a coating with perforations evenly distributed;
Fig. 2c is a top view of a medical implant having a coating with perforations, wherein the density of perforations in some areas is higher than the density of perforations in other areas;
Fig. 2d is a top view of a medical implant having a coating with perforations, wherein a diameter of perforations in some areas is larger than a diameter of perforations in other areas;
Fig. 3 is a lateral view of a medical implant, which has been provided with a coating at both ends;
Fig. 4 is a top view of a medical implant with a coating covering only a part of the medical implant in a radial direction;
Fig. 5 is a lateral view of a medical implant in an expanded shape;
Fig. 6 is a lateral view of a medical implant in a contracted shape;
Fig. 7 is a top view of a medical implant, provided with a coating, wherein the coating forms a pattern;
Fig. 8 is a lateral view of a medical implant being coated by dipping;
Fig. 9 is a lateral view of a medical implant being coated by spraying; and
Fig. 10 is a schematic sketch of a medical implant being coated by a process, such as electro-spinning or Nano-spinning.

### DESCRIPTION OF THE PREFERRED EMBODIMENTS

Specific embodiments of the disclosure now will be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

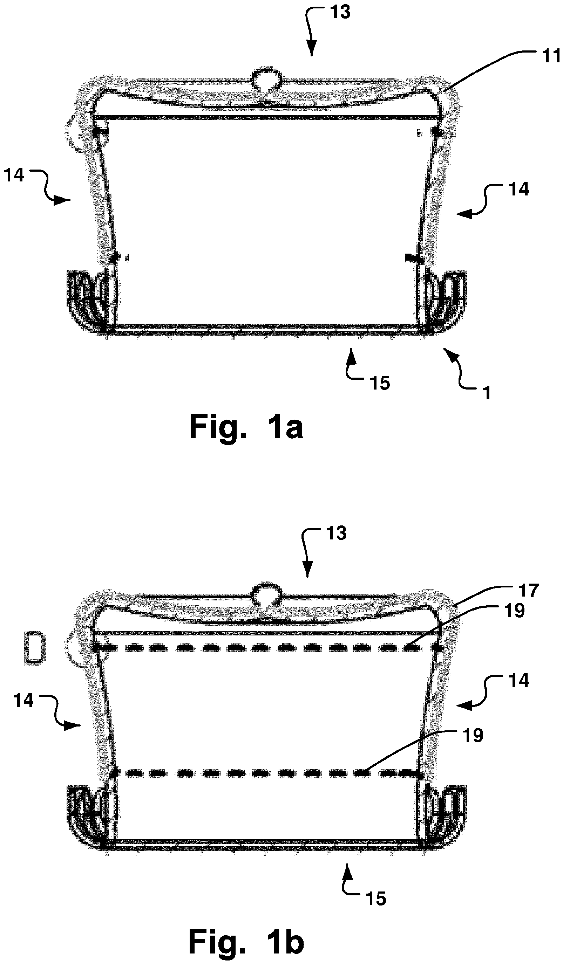

The following description focuses on an embodiment of the present disclosure applicable to medical implants and in particular to a left atrial appendage (LAA) occluder. However, it will be appreciated that the disclosure is not limited to this application but may be applied to many other medical implants including for example Filters, Stents, Vascular Occluders, Products for treatment of aneurysm, Plugs and Occlusion systems for other applications, such as atrial septal defect (ASD) occluders, Patent foramen ovale (PFO) occluders, paravalvular leakage (PLD) occluders and ventricular septal defect (VSD) occluders.
In an embodiment of the disclosure according to Fig. 1a, a medical implant 1 is provided with a coating 11. The coating 11 has been applied to an external surface of the medical implant 1. The medical implant has been coated on the outside by a method, in which the medical implant 1 has been dipped in a solution with a specific viscosity, while the medical implant 1 was in an expanded shape. By applying the coating 11 to the medical device 1, while the medical device 1 is in an expanded shape, the coating 11 is free of tension, which advantageously avoids pre-mature fatigue thereof and thus allows a reliable ingrowth. Application of a coating 11 to a medical implant 1, while the medical device 1 is in an expanded shape, may also be advantageous for other reasons, such as the fact that the medical implant 1 can be made very flexible and that a particularly large expansion/contraction ratio, i.e. a ratio of a size or diameter of the medical implant 1 in its expanded shape and the size or diameter of the medical implant 1 in its contracted shape, can be obtained for the medical implant 1.

By making sure that the solution has a specific viscosity, the coating can be made non-fibrous. The specific viscosity is a viscosity, which takes on a value, which is in an interval, where the solution for the coating is non-fibrous or not fibrous. Thus, the coating will be made fibrous. This may be advantageous, since e.g. a lower friction towards a catheter is achieved. By having a lower friction towards the walls of a delivery catheter, the delivery is facilitated and made smoother, i.e. the medical implant slides or glides more smoothly through the delivery catheter. In one embodiment only one end 13, and not the side 14 of the medical implant 1, which side 14 encircles the medical implant 1, is dipped into the solution. In another embodiment, the end 13 and part of or the whole side 14 are dipped into the solution, so as to be provided with coating. Thereby, a large portion of the medical implant 1 is covered with the coating 11. In yet another embodiment, only the ends of the medical implant 1 are dipped into the solution, i.e. the end 13 and the other end 15 are dipped into the solution, but the side 14 is not dipped into the solution. Thereby, the medical implant is covered at both ends. This can be done by first dipping the end 13 into the solution, then retracting the medical implant 1 from the solution. Thereafter, the medical implant is turned around and with the other end 15 facing the solution, the medical device is again dipped into the solution. A coating applied to the medical device 1 provides for an improved occlusion, improved sealing of a defect, such as a heart defect, an improved endothelialization and/or for slowing down the blood flow through the defect.

In an embodiment according to Fig. 1b, a medical implant 1 is instead provided with a non-fibrous membrane 17 externally, i.e. on the outside of the medical implant 1. Such a non-fibrous membrane 17 may be sewed onto the medical implant 1 by stitching it onto the medical implant 1 along the medical implant's circumference with stitches 19. In some embodiments, the non-fibrous membrane 17 only covers one end 13, and not the side 14, of the medical implant 1. In another embodiment, the end 13 and the side 14 are provided with a non-fibrous membrane 17. In yet another embodiment, only the ends of the medical implant 1 are provided with a non-fibrous membrane 17, i.e. the end 13 and the other end 15 are provided with a non-fibrous membrane 17, whereas the side 14 is not provided with any non-fibrous membrane 17. Thereby, the medical implant is covered at both ends 13, 15. A non-fibrous membrane 17 applied to the medical device 1 provide for an improved occlusion, improved sealing of a defect, such as a heart defect, an improved endothelialization and/or for slowing down the blood flow through the defect. However, a coating 11 offers an advantage over the non-fibrous membrane 17, since there is no stitching, no sewing or even any clips needed for attaching and/or affixing the coating to the medical implant 1. Thus, the applying of a coating 11 instead of a non-fibrous membrane 17 may offer the advantage of providing easier and cheaper manufacturing. When compared to providing membranes or patches inside the medical implant 1, this advantage may be even greater, since the applying of membranes or patches inside such a medical implant 1 is even more time-consuming and complicated than just applying a non-fibrous membrane on an outside of the medical implant 1, because the membranes or patches has to first be put inside the medical implant 1 and then sewed or stitched onto the medical implant, while being inside the medical implant 1.

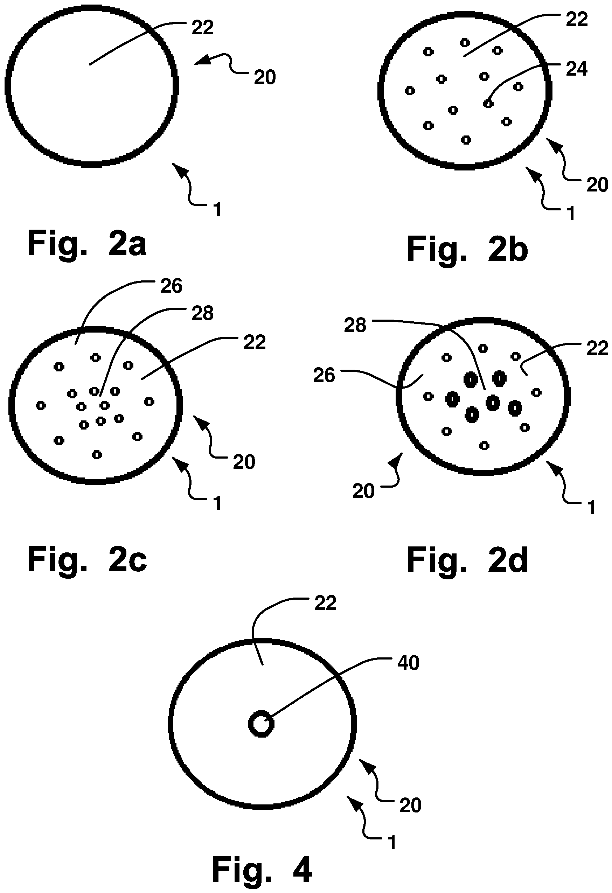

According to an embodiment, depicted in fig. 2a, one end 20 of the medical implant 1 is completely covered with a coating 22. Thus, an improved occlusion, an improved sealing of a defect, such as a heart defect, an improved endothelialization and/or a slowing down of the blood flow through the defect is achieved. However, in order to provide some body liquid to pass through the medical implant 1 once implanted, the coating may be provided with perforations or microperforations. This is shown in fig. 2b, which depicts a situation where one end 20 of the medical implant 1 has been covered with a coating 22 and where the coating has been perforated, i.e. provided with perforations 24 or microperforations. Such perforations or microperforations may be provided by a process, such as mechanical perforation or laser perforation, i.e. laser cutting. The use of laser perforation offers the advantage of a better consistency of the hole size, i.e. the perforation size, than the use of mechanical perforation. By providing the coating 22 with perforations or microperforations, an initial controllable body liquid retention is enabled. Furthermore, the integration of the medical implant 1 may be enhanced and/or facilitated by the use of such perforations 24 or microperforations, since the body liquid is allowed to enter into the interior of the medical implant 1. A limited blood flow may actually pass through the medical implant 1 after implantation. However, this limited blood flow will gradually decline upon blood coagulation and/or endothelialization of the implanted medical implant. Thus, by the use of perforations 24 or microperforations, the occlusion is not abrupt, but formed gradually over time.

In one embodiment, the perforations 24 or microperforations of the coating 22 are uniformly distributed over the area of the coating 22. However, in other embodiments, the perforations 24 or microperforations are randomly distributed. In yet another embodiment, depicted in fig. 2d, a first central area 28 of the coating 22, corresponding to a first area of the medical implant is provided with perforations of a larger size, such as a diameter, than perforations of a second peripheral area 26 of the coating 22, corresponding to a second area of the medical implant 1, so that the inflow to different areas is controlled. As an alternative, the first central area 28 of the coating 22, corresponding to a first area of the medical implant 1, is provided with a higher number of perforations or a higher density of perforations than a second peripheral area 26 of the coating 22, corresponding to a second area of the medical implant, so that the inflow to different areas is controlled. This is depicted in fig. 2c.

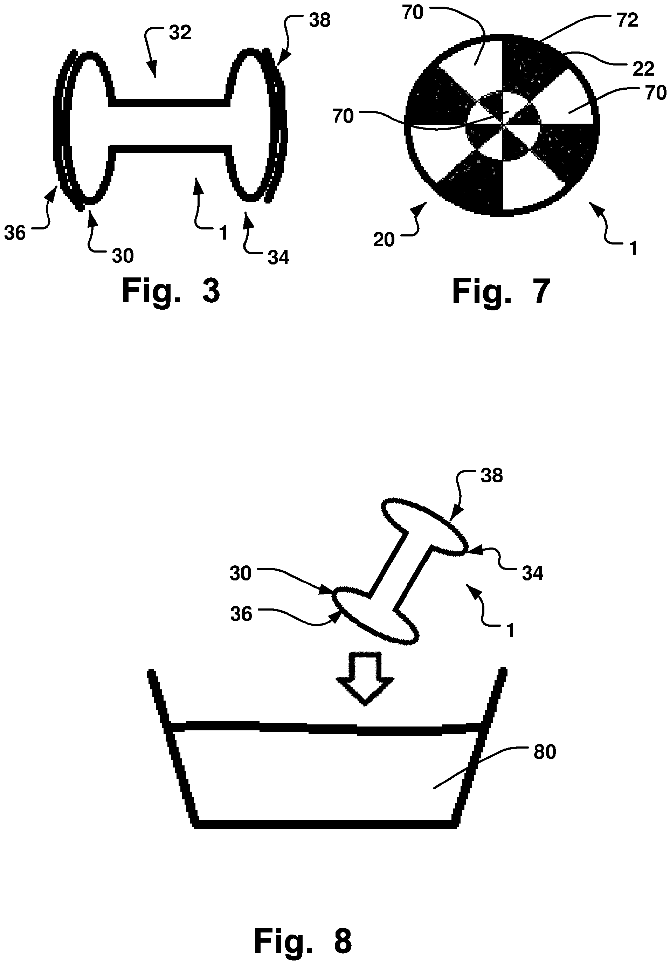

In fig. 3, another kind of medical implant 1 or occluder is shown. This medical implant 1 comprises a first disc-shaped section 30, a tubular middle section 32 and a second disc-shaped section 34. In this embodiment, the one depicted in fig. 3, only the ends of the medical implant 1 are coated or provided with non-fibrous membranes, i.e. the outer end side 36 of the first disc-shaped section 30 and the outer end side 38 of the second disc-shaped section 34 are coated or provided with non-fibrous membranes. The application of a coating can e.g. be performed by dipping both outer end sides 36, 38 into a solution, with a specific viscosity. Thereby, the medical implant is covered at both ends. In another embodiment, only the outer end side 36 of the first disc-shaped section 30 is provided with a coating 22. In yet another embodiment, the full length of the medical implant 1 is provided with a coating 22. In some embodiments, the tubular middle section 32 is provided with a coating 22, whereas the disc-shaped sections 34, 36 are not provided with a coating.

In yet another embodiment, depicted in fig. 4, a coating 22 or non-fibrous film membrane is arranged at an end 20 of a medical implant 1 so as to obtain an inflow of blood, into the inner of the medical implant 1, after implantation and thus in an expanded shape, from a distal end of the medical implant 1 for enhancing integration of the medical implant with surrounding blood upon clotting thereof. This can e.g. be achieved by providing the end 20 with a coating 22, which covers substantially the whole end 20, but does not cover the section 40 of the end 20. Thus, an inflow of blood, into the inner of the medical implant 1 can be obtained through the section 40. The section 40 may be centred or situated at any other position at the end 20. As an alternative, the section 40 may instead by surrounding the coating 22, i.e. the coating 22 is applied only to a central portion of the end 20.

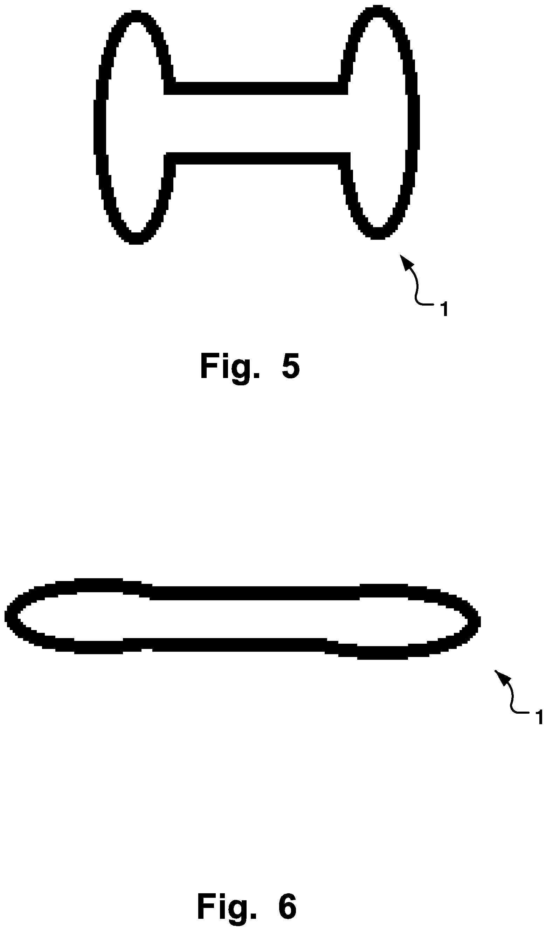

Fig. 5 shows a medical implant 1 in an expanded shape. This is the shape the medical implant 1 preferably has after being implanted. This is also the shape, the medical implant resiliently returns to, i.e. it can also be called the relaxed shape. However, in order to deliver a medical implant 1 into a target site inside a mammal body, the medical implant 1 needs to be put through a narrow delivery catheter. In order for the medical implant 1 to fit into a narrow delivery catheter, the medical implant 1 will have to take on another shape. This other shape is here called the contracted shape. It could also be called a delivery shape. The medical implant in its contracted shape can be seen in fig. 6. The coating 22 is in one embodiment applied to the medical implant 1, while the medical implant 1 is in the contracted shape. Thereby, the coating 22 will be prone to contribute to force the medical implant into its contracted shape and thus provide for facilitation of the delivery of the medical implant 1 through a catheter.

Fig. 7 is a top view of a medical implant, provided with a coating 22, wherein the coating 22 forms a pattern. Such a pattern can be any pattern, which is advantageous for control of a desired flow pattern through the medical implant 1 upon implantation. In the embodiment according to fig. 7, the medical implant 1 is provided with a coating 22 in some sections, i.e. one section 72 of the end portion 20 of the medical implant 1 is provided with a coating 22, whereas all adjacent sections 70 are not provided with a coating and likewise all sections being adjacent to a non-coated section 70 are provided with a coating 22. By the use of such a pattern of covered sections 72 or cells an efficient control of a desired flow pattern through the medical implant 1 upon implantation is established. The sections or cells may be large and thus cover large portions of the medical implant or as small as a gap between adjacent strands of the mesh, which makes up the medical implant 1. The pattern may be formed be first applying a coating 22 by a method, such as dipping the medical implant 1 into a solution, and thereafter removing parts of the coating 22 so as to form a pattern of coating 22.

A step of a method of producing a medical implant for occluding an opening in a body is shown in Fig. 8, which is a lateral view of a medical implant being coated by dipping. In some embodiments, such a method comprises producing a body mesh of strands forming a plurality of adjacent cells delimited by the strands. Some or all of these cells may be provided with a coating. Therefore, in some embodiments, the method further comprises applying a polymer, such as polyurethane, polytetrafluoroethylene (PTFE) or expanded polytetrafluoroethylene (ePTFE), to at least part of an external surface of the medical implant 1, such as the outer end side 36 of the first disc-shaped section 30 of the medical implant 1. The use of e.g. PTFE or ePTFE may be especially advantageous, since these materials may provide for low friction. The polymer may be applied to the medical implant 1 by e.g. dipping, spraying, electro-spinning, electro-spraying or Nano-spinning. As another alternative, instead of coating the medical implant 1, a non-fibrous film membrane may be sewed onto the external surface of the medical implant 1. Such a non-fibrous film membrane may be manufactured by applying a film on a substrate and then removing the substrate. E.g. a film can be applied by dipping a substrate into a solution and thereafter removing the substrate. As an alternative, a substrate can be sprayed and thereafter removed from the coating formed by the spray, so that a non-fibrous film membrane is obtained.

In figure 8, the process of dipping a medical implant into a solution 80 of a specific viscosity is shown. Thus, the polymer is applied to the medical implant 1 by dipping the medical implant 1 into a solution 80 of a specific viscosity, so that a non-fibrous coating is applied and affixed to an external surface of the medical implant 1. In one embodiment only an outer end side 36 of a first disc-shaped section 30 of the medical implant is dipped into the solution. In another embodiment, the outer end side 36 of the first disc-shaped section 30 and an outer end side 38 of a second disc-shaped section 34 of the medical implant 1 are dipped into the solution. In other embodiments, further parts of the medical implant 1 may be dipped into the solution. As an example, the side 14 (shown in fig. 1a) can be dipped into the solution. As another example, substantially the whole medical implant 1 may be dipped into the solution. Which parts of the medical implant 1 that are dipped into the solution may depend on what kind of medical device 1 is to be applied with coating, i.e. it may depend on whether the medical implant is e.g. an atrial septal defect (ASD) occluder, a Patent foramen ovale (PFO) occluder, a paravalvular leakage (PLD) occluder, a ventricular septal defect (VSD) occluder or some other medical implant.

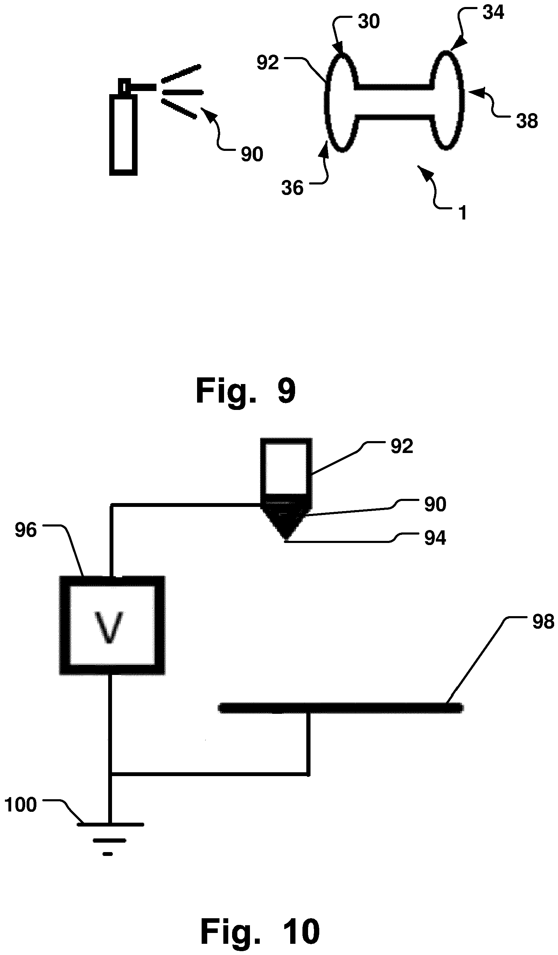

Alternatively, the coating can be applied to the medical implant 1 by spraying the medical implant with a spray 90, which is of a specific viscosity, so that a non-fibrous coating 92 is applied and affixed to an external surface of the medical implant 1. This alternative is shown in Fig. 9, which is a lateral view of a medical implant 1 being coated by spraying. In one embodiment only an outer end side 36 of a first disc-shaped section 30 of the medical implant is sprayed. In another embodiment, the outer end side 36 of the first disc-shaped section 30 and an outer end side 38 of a second disc-shaped section 34 of the medical implant 1 are sprayed. In other embodiments, further parts of the medical implant 1 may be sprayed. As an example, the side 14 (shown in fig. 1a) can be sprayed. As another example, substantially the whole medical implant 1 may be sprayed. Which parts of the medical implant 1 that are sprayed may depend on what kind of medical device 1 is to be applied with coating, i.e. it may depend on whether the medical implant is e.g. an atrial septal defect (ASD) occluder, a Patent foramen ovale (PFO) occluder, a paravalvular leakage (PLD) occluder, a ventricular septal defect (VSD) occluder or some other medical implant.

Other alternatives of applying a coating to the medical implant 1 are e.g. electro-spinning, electro-spraying or Nano-spinning. Fig. 10 is a schematic sketch of a medical implant being coated by a process, such as electro-spinning or Nano-spinning. In the figure, a polymer or composite solution 90 is contained in a syringe pump 92. The syringe pump 92 comprises a spinneret, such as a hypodermic syringe needle or a metallic needle, which is connected to a high-voltage direct current power supply 96. The direct current power supply 96 is also connected to ground 100 and on the ground side, a collector 98 is connected to the direct current power supply 96. The medical implant 1 to be coated would typically be placed in connection with the collector 98. The polymer solution 90 is loaded into the syringe 92 and extruded, as droplets, from the tip of the needle 94 at a constant rate by the syringe pump 92. A sufficiently high voltage must then be applied to the droplets, so that the droplets become charged. Since electrostatic repulsion counteracts the surface tension, the droplets are stretched: At a critical point, a stream of liquid erupts from the surface. This point of eruption is known as the Taylor cone. If the molecular cohesion of the liquid is sufficiently high, stream breakup does not occur and a charged liquid jet is formed. Alternatively, if stream breakup occurs, the droplets are instead electro-sprayed.

As the jet dries in flight, the mode of current flow changes from ohmic to convective as the charge migrates to the surface of the strand. The jet is then elongated by a whipping process caused by electrostatic repulsion initiated at small bends in the strand, until it is finally deposited on the grounded collector. The elongation and thinning of the strand resulting from this bending instability leads to the formation of uniform strands. Such uniform strands may have nanometer-scale diameters.

In some embodiments, a method of producing a medical implant for occluding an opening in a body comprises producing a body mesh of strands forming a plurality of adjacent cells delimited by the strands. The producing of a body mesh of strands may be performed by intertwining strands along a length of a 3D fabric for forming a primary 3D fabric structure. The intertwining may be noncontinuous, such as interrupted along the length, for forming a secondary structure of the 3D fabric without intertwining. A round braiding machine may be used for forming the primary and secondary fabric structures. In one embodiment, the method may comprise connecting a first end of a strand to a bobbin of a round braiding machine with a plurality of bobbins and a second end of the strand to a diametrically opposing bobbin of the round braiding machine for a plurality of strands and arranging middle sections of the plurality of strands in a fixed sequence over a braiding head in a crisscrossed manner. It may further comprise starting a braiding procedure, halting the braiding procedure, placing a crown-shaped holder for holding a plurality of strand loops at the braiding head, bending remaining strand sections individually in the middle sections in order to form strand loops, introducing the remaining strand sections into a space between the braiding head and the crown-shaped holder from below, placing the strand loops over pins of the crown-shaped holder, routing the strand ends to the bobbins, attaching the strand ends to the bobbins, placing a ring on top of the crown-shaped holder for fixation of the strand loops, continuing the braiding procedure until an intended strand length has been braided, detaching the strand ends from the bobbins, attaching the strand ends to the ring with fixation means and/or treating the braided material, the ring and the crown-shaped holder thermally for shaping of the medical implant. It may also comprise welding the strand ends together, by at least partly melting a length of the plurality of strands to form a defined ball pivot. Thereby, shaping of loops can be made accurately, fastly and/or easily.

The medical implant 1 may also in one embodiment be constructed so that the ends of the medical implant 1 folds inwards for delivery, i.e. when the medical implant 1 is in its contracted shape, the coating 22 is on the inside and covered and/or protected by the sides of the medical implant 1, which sides are close to or touching the inside wall of a delivery catheter, in which it is delivered. The ends of the medical implant 1 are in this embodiment somewhat conically shaped or funnel-shaped, so that the medical implant 1 folds into its contracted shape with its coated ends covered on the outside with the sides of the medical device 1. The two disc-shaped sections 30, 34 of the medical device 1 may also be cupped away from each other.

The present disclosure has been described above with reference to specific embodiments. However, other embodiments than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

## Claims

1. A medical implant, such as an atrial septal defect (ASD) occluder, a Patent foramen ovale (PFO) occluder, a paravalvular leakage (PLD) occluder or a ventricular septal defect (VSD) occluder, for occluding an opening in a body, wherein said medical implant (1) comprises
a body mesh of strands forming a plurality of adjacent cells delimited by said strands,
said body mesh having an external surface, and
a coating (22) or a non-fibrous film membrane (17) covering said external surface for at least partly restricting a fluid flow through a structural tissue defect, such as a defect in the heart.

2. The medical implant of claim 1, wherein said coating (22) is provided as a non-fibrous film membrane (17).

3. The medical implant of claim 2, wherein said coating (22) or non-fibrous film membrane (17) is provided with perforations (24) or microperforations for enabling an initial controllable fluid retention.

4. The medical implant of claim 3, wherein a first area of said coating (22), corresponding to a first area of said medical implant (1) is provided with perforations (24) of a larger size, such as a diameter, than perforations (24) of a second area of said coating (22), corresponding to a second area of said medical implant (1) so that the inflow to different areas is controlled or wherein a first area of said coating (22), corresponding to a first area of said medical implant (1) is provided with a higher number of perforations (24) than a second area of said coating (22), corresponding to a second area of said medical implant (1) so that the inflow to different areas is controlled.

5. The medical implant of claim 4, wherein said coating (22) or non-fibrous film membrane (17) covers substantially the full diameter of both ends of said medical implant (1).

6. The medical implant of claim 4, wherein said coating (22) or non-fibrous film membrane (17) covers substantially a full expanded diameter of said medical implant (1) and/or wherein said coating (22) or non-fibrous film membrane (17) covers substantially a full length of said medical implant (1).

7. The medical implant of claim 4, wherein said coating (22) or non-fibrous film membrane (17) only covers a portion of a full expanded diameter of said medical implant (1) and/or wherein said coating (22) or non-fibrous film membrane (17) only covers a portion of a full length of said medical implant (1).

8. The medical implant according to any of claims 1-7, wherein said coating (22) or non-fibrous film membrane (17) is arranged so as to obtain an inflow of blood, into the inner of said medical implant (1) in an expanded shape, from a distal end of said medical implant (1) for enhancing integration of said medical implant (1) with surrounding blood upon clotting thereof.

9. The medical implant according to any of claims 1-8, wherein said coating (22) or non-fibrous film membrane (17) is applied to said medical implant (1), while said medical implant (1) is in an expanded shape.

10. The medical implant according to any of claims 1-8, wherein said coating (22) or non-fibrous film membrane (17) is applied to said medical implant (1), while said medical implant (1) is in a contracted shape.

11. The medical implant according to any of claims 1-9, wherein said medical implant (1) is covered with said coating (22) so that a pattern of covered cells is established for efficient control of a desired flow pattern upon implantation.

12. The medical implant of claim 1 wherein said coating (22) or said non-fibrous film membrane (17) is made of a material, such as Polyurethane (PU), Polytetrafluoroethylene (PTFE) or Expanded Polytetrafluoroethylene (ePTFE).

13. A method of producing a medical implant for occluding an opening in a body, said method comprising:
producing a body mesh of strands forming a plurality of adjacent cells delimited by said strands; and
applying a polymer to at least part of an external surface of said medical implant (1), wherein said polymer is applied to said medical implant (1) by dipping, spraying, electro-spinning, electro-spraying, Nano-spinning or by sewing a non-fibrous film membrane (17) onto said external surface of said medical implant (1).

14. The method of claim 13, wherein said polymer is applied to said medical implant (1) by dipping said medical implant (1) into a solution of a specific viscosity so that a non-fibrous coating (22) is applied and affixed to an external surface of said medical implant (1).

15. The method of claim 13, wherein said polymer is applied to said medical implant (1) by spraying said medical implant (1) with a spray having a specific viscosity so that a non-fibrous coating (22) is applied and affixed to an external surface of said medical implant (1).
